# EUROPEAN PATENT SPECIFICATION

(11) **EP 1 015 593 B1**
(45) Date of publication and mention of the grant of the patent: **21.05.2003**
(21) Application number: 98944071.4
(22) Date of filing: 21.09.1998
(51) Int. Cl.: C12N 15/36, C12N 15/62, C12N 15/70, C12N 1/21, C07K 16/08, A61K 38/16, A61K 39/29, A61K 48/00

(54) **HEPATITIS B VIRUS POLYPEPTIDES**
HEPATITIS B VIRALE POLYPEPTIDE
POLYPEPTIDES DU VIRUS DE L'HEPATITE B

(30) Priority: 19.09.1997 GB 9720033
(43) Date of publication of application: 05.07.2000
(73) Proprietor: Celltech Pharma Europe Limited, Slough, Berkshire SL1 3WE (GB)
(72) Inventor: CHATFIELD, Steven, Neville, Du Cane Road London W12 0NN (GB)
(74) Representative: Campbell, Patrick John Henry
(86) International application number: GB9802852
(87) International publication number: WO99015671

(56) References cited:
- EP-A- 0 389 983
- WO-A-88/10300
- WO-A-94/01132
- WO-A-94/03615
- WO-A-95/04151
- WO-A-95/20665
- SHI C -H ET AL: "Gene fusion of cholera toxin B subunit and HBV PreS2 epitope and the antigenicity of fusion protein" VACCINE, vol. 13, no. 10, July 1995, page 933-937 XP004057512
- KHAN C. ET AL.: "Construction, expression, and immunogenecity of the Schistosoma mansoni P28 glutathione S-transferase as a genetic fusion to tetanus toxin fragment C in a live Aro attenuated vaccine strain of Salmonella" PNAS,U.S.A., vol. 91, no. 23, 1994, pages 11261-11265, XP002090995

## Description

### Field of the invention

This invention relates to fusion polypeptides derived from the surface antigen of hepatitis B virus and their use in vaccine compositions.

### Background to the invention

Hepatitis B infection and consequential diseases which include chronic liver disease, cirrhosis and hepatocellular carcinoma are a major health problem throughout the world. Systematic vaccination of individuals at risk of exposure to the virus has been the main method of controlling infection. The first hepatitis B virus (HBV) vaccine was manufactured by the purification and inactivation of HBV surface antigen (HBsAg) obtained from the plasma of chronic carriers. This was soon followed by the production of HBsAg using recombinant DNA techniques. However, a significant proportion of individuals do not mount antibody responses to the HBsAg present in vaccine preparations and it is considered that these individuals remain susceptible to infection with HBV.

### Summary of the Invention

The present invention provides a polypeptide comprising tetanus toxin fragment C, or a fragment thereof, fused to the pre-S1 region of hepatitis B virus (HBV), or a fragment thereof, and/or the pre-S2 region of HBV or a fragment thereof.

Preferably said fragment of the pre-S1 region and pre-S2 region comprises at least 5 amino acids, more preferably at least 6 amino acids, most preferably 10, 15 or 20 amino acids.

The tetanus toxin fragment C or fragment thereof may be fused to the pre-S1 region, or fragment thereof, of hepatitis B virus (HBV) or the pre-S2 region of HBV, or fragment thereof, via a "hinge" linker region. Similarly, where both a fragment of the pre-S1 region and a fragment of the pre-S2 region are present, they may be joined together by a "hinge" linker region.

The present invention further provides a polynucleotide encoding a polypeptide of the invention

The present invention also provides vectors comprising a polynucleotide encoding a polypeptide of the invention operably linked to a regulatory sequence. Preferably the regulatory sequence allows expression of the polypeptide in a host cell. Typically the host cell is a bacterium, which may be attenuated or a cell of an animal, more preferably a mammal, including primates and humans.

The polypeptides, polynucleotides and vectors and host cells of the present invention may be used in the prevention or treatment of hepatitis B viral infections. Thus, in a further aspect, the present invention provides a vaccine composition comprising a polypeptide, polynucleotide or vector of the invention together with a pharmaceutically acceptable carrier or diluent. The vaccine composition may comprise attenuated bacterium transformed with a polynucleotide of the invention. It may be preferred to use the polypeptides of the invention in combination with the active constituents of other HBV vaccine compositions to increase their effectiveness. Thus the vaccine composition of the invention preferably further comprises, for example, the polypeptide components of the HBV vaccine described in WO88/10301 (i.e. the S, S+pre-S2 and S+amino acids 20 to 47 of pre-S1 antigenic components of both subtypes *adw* and *ayw*).

The polypeptides of the invention may also be used to induce antibody responses in non-human animals. The resulting antibodies may be polyclonal antibodies or monoclonal antibodies, or fragments thereof. These antibodies may be used in a method of treating HBV infection in a human or animal.

In addition to the potential therapeutic uses of the polypeptides, polynucleotides, vectors and antibodies of the invention, they may also be used as tools to determine, for example, antigenic determinants within the pre-S1 and/or pre-S2 regions of the HBV surface antigen (HBsAg). Both regions are believed to play important roles in augmenting anti-HBsAg responses which prevent attachment of the virus to hepatocytes and elicit antibodies which are effective in viral clearance, stimulating cellular immune responses and circumventing genetic non-responsiveness to the S region alone.

### Detailed Description of the Invention

### A. Polypeptides

The polypeptides of the invention comprise tetanus toxin fragment C or an epitope containing fragment thereof fused to a fragment of the pre-S1 region of hepatitis B virus (HBV) and/or a fragment of the pre-S2 region of HBV.

The structural gene for tetanus toxin has been cloned and sequenced (Fairweather et al. (1986), *J*. *Bacteriol*, **165**, p21-27). Fragment C is a 50 kDa polypeptide generated by papain cleavage and comprises or substantially corresponds to the 451 amino acids at the C-teminus. Fragments of tetanus toxin fragment C that contain epitopes may also be used in the polypeptides of the invention. These fragments will comprise at least 5 or 6 amino acids, preferably at least 10 amino acids, more preferably at least 15, 20, 50 or 100 amino acids. Particularly preferred fragments include from about amino acids 80 to 180, which is a good B-cell epitope, and from about amino acids 83 to 103 and 409 to 420 which are good T-cell epitopes (numbering assumes that amino acid 1 of fragment C is amino acid 864 of the complete tetanus toxin).

Preferably the fragment of the pre-S1 region and pre-S2 region comprises at least 5 amino acids, more preferably at least 6 amino acids, most preferably 10, 15 or 20 amino acids. The fragment may include, for example, amino acids 1 to 19, 20 to 39,40 to 59, 60 to 79, 80 to 99 or 100 to 119 of pre-S1, or I to 19,20 to 39 or 40 to 55 of pre-S2. Suitable fragments which may be used in the polypeptides of the invention are described in the Examples. Particularly preferred fragments include amino acids 20 or 21 to 47 of pre-S 1 (the hepatocyte binding site) and amino acids 1 to 26 and 14 to 32 of pre-S2.

Further, the amino acid sequence of tetanus toxin fragment C and the fragments of the pre-S1 and pre-S2 regions can be modified to provide polypeptides of the invention. For example, this may be carried out to enhance the immunogenicity of the polypeptides of the invention. Amino acid substitutions may be made, for example from 1, 2 or 3 to 10, 20 or 30 substitutions provided that the modified polypeptide retains epitopes.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The tetanus toxin fragment C may be fused to the fragment of the pre-S 1 region of hepatitis B virus (HBV) or the fragment of the pre-S2 region of HBV via a "hinge" linker region. Similarly, where both a fragment of the pre-S1 region and a fragment of the pre-S2 region are present, they may be joined together by a "hinge" linker region.

The "hinge" linker region is a region designed to promote the independent folding of both the tetanus toxin fragment C, fragment of the pre-S1 region and the fragment of the pre-S2 region by providing both spatial and temporal separation between the domains.

The hinge region typically is a sequence encoding a high proportion of proline and/or glycine amino acids. The hinge region may be composed entirely of proline and/or glycine amino acids. The hinge region may comprise one or more glycine-proline dipeptide units. In the alternative the hinge region may comprise the carboxy terminal of tetanus toxin fragment C.

The hinge region may, for example, contain up to about fifteen amino acids, for example at least 4 and preferably from 6 to 14 amino acids, the number of amino acids being such as to impart flexibility between the different polypeptide domains.

In one embodiment, the hinge region can correspond substantially to the hinge domain of an antibody immunoglobulin. The hinge regions of IgG antibodies in particular are rich in prolines (T.E. Michaelson *et al*. (1977) J. Biol. Chem. **252**, p883-9), which are thought to provide a flexible joint between the antigen binding and tail domains.

Other amino acids may be substituted for glycine, particularly those without bulky side-chains, such as alanine, serine, asparagine and threonine.

In one preferred embodiment, the hinge region is a chain of four or more amino acids defining the sequence:

- [X]p-Pro-[Y]q-Pro-[Z]r-

wherein Pro is proline, X and Y are each glycine, or an amino acid having a non-bulky side chain; Z is any amino acid; p is a positive integer; q is a positive integer of from one to ten; and r is zero or a positive integer greater than zero.

### B. Polynucleotides and vectors.

Polynucleotides of the invention comprise nucleic acid sequences encoding the polypeptides of the invention. Polynucleotides of the invention may comprise DNA or RNA. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the in vivo activity or life span of polynucleotides of the invention.

Preferred polynucleotides of the invention also include polynucleotide encoding any of the polypeptides of the invention described above. It will be understood by a skilled person that numerous different polynucleotides can encode the same polypeptide as a result of the degeneracy of the genetic code.

Polynucleotides of the invention comprise can be incorporated into a recombinant replicable vector. The vector may be used to replicate the nucleic acid in a compatible host cell. Thus in a further embodiment, the invention provides a method of making polynucleotides of the invention by introducing a polynucleotide of the invention into a replicable vector, introducing the vector into a compatible host cell, and growing the host cell under conditions which bring about replication of the vector. The vector may be recovered from the host cell. Suitable host cells include bacteria such as *E. coli*, yeast, mammalian cell lines and other eukaryotic cell lines, for example insect Sf9 cells.

Preferably, a polynucleotide of the invention in a vector is operably linked to a regulatory sequence that is capable of providing for the expression of the coding sequence by the host cell, i.e. the vector is an expression vector. The term "operably linked" refers to a juxtaposition wherein the components described are in a relationship permitting them to function in their intended manner. A regulatory sequence "operably linked" to a coding sequence is ligated in such a way that expression of the coding sequence is achieved under condition compatible with the control sequences.

Such vectors may be transformed or transfected into a suitable host cell as described above to provide for expression of a polypeptide of the invention. This process may comprise culturing a host cell transformed with an expression vector as described above under conditions to provide for expression by the vector of a coding sequence encoding the polypeptides. The expressed polypeptides may be recovered *in vitro*. Host cells transformed to provide stable expression of the polypeptides may also be used *in vivo*. For example, a host cell such as an attenuated bacterium transformed to express a polypeptide of the invention may be used as a vaccine. The attenuated bacterium may be selected from *Salmonella, Bordetella, Vibrio*, *Haemophilus, Neisseria* and *Yersinia*. More preferably the attenuated bacterium is an enterobacteria such as *E.coli* or *Salmonella,* such as, *S.typhis, S.typhimurium* or *S.enteritidis*.

The vectors may be for example, plasmid or virus vectors provided with an origin of replication, optionally a promoter for the expression of the said polynucleotide and optionally a regulator of the promoter. The vectors may contain one or more selectable marker genes, for example an ampicillin resistance gene in the case of a bacterial plasmid or a neomycin resistance gene for a mammalian vector. Vectors may be used *in vitro*, for example for the production of RNA or used to transfect or transform a host cell. The vector may also be adapted to be used *in vivo*, for example in a method of gene therapy.

Promoters/enhancers and other expression regulation signals may be selected to be compatible with the host cell for which the expression vector is designed. For example, prokaryotic promoters may be used, in particular those suitable for use in E. coli strains (such as *E*. *coli* HB101). In a particularly preferred embodiment of the invention, a promoter whose activity is induced in response to a change in the surrounding environment, such as anaerobic conditions is used. Preferably an htrA or nirB promoter may be used. These promoters may be used in particular to express the polypeptides in attenuated bacterium for example for use as a vaccine. When expression of the polypeptides of the invention in carried out in mammalian cells, either *in vitro* or *in vivo*, mammalian promoters may be used. Tissues-specific promoters, for example hepatocyte cell-specific promoters, may also be used. Viral promoters may also be used, for example the Moloney murine leukaemia virus long terminal repeat (MMLV LTR), the promoter rous sarcoma virus (RSV) LTR promoter, the SV40 promoter, the human cytomegalovirus (CMV) IE promoter, herpes simplex virus promoters or adenovirus promoters. All these promoters are readily available in the art.

### C. Administration

The polypeptides of the invention may be administered by direct injection. Preferably the polypeptides are combined with a pharmaceutically acceptable carrier or diluent to produce a pharmaceutical composition. Suitable carriers and diluents include isotonic saline solutions, for example phosphate-buffered saline. The composition may be formulated for parenteral, intramuscular, intravenous, intranasal, subcutaneous, intraocular or transdermal administration. Typically, each polypeptide is administered at a dose of from 0.01 to 30 µg/kg body weight, preferably from 0.1 to 10 µg/kg, more preferably from 0.1 to 1 µg/kg body weight. It is also possible to use antibodies prepared using the polypeptides of the invention, as described below, in treating or preventing HBV infection. Neutralising antibodies, or fragments thereof which retain specificity for HBV antigens, can be administered in a similar manner to the polypeptides of the invention.

The polynucleotides of the invention may be administered directly as a naked nucleic acid construct, preferably further comprising flanking sequences homologous to the host cell genome. When the expression cassette is administered as a naked nucleic acid, the amount of nucleic acid administered is typically in the range of from 1 µg to 10 mg, preferably from 100 µg to 1 mg.

Uptake of naked nucleic acid constructs by mammalian cells is enhanced by several known transfection techniques for example those including the use of transfection agents. Example of these agents include cationic agents (for example calcium phosphate and DEAE-dextran) and lipofectants (for example lipofectam™ and transfectam™). Typically, nucleic acid constructs are mixed with the transfection agent to produce a composition.

Preferably the polynucleotide or vector of the invention is combined with a pharmaceutically acceptable carrier or diluent to produce a pharmaceutical composition. Suitable carriers and diluents include isotonic saline solutions, for example phosphate-buffered saline. The composition may be formulated for parenteral, intramuscular, intravenous, subcutaneous, intraocular or transdermal administration.

The routes of administration and dosages described are intended only as a guide since a skilled practitioner will be able to determine readily the optimum route of administration and dosage for any particular patient and condition.

### D. Preparation of Vaccines

Vaccines may be prepared from one or more polypeptides of the invention. They may also include one or more immunogenic HBV polypeptides, for example immunogenic HBV polypeptides known in the art. Thus a vaccine of the invention may comprise one or more polypeptides of the invention and optionally, one or more polypeptides selected from HBV S, pre-S1, pre-S2 polypeptides and immunogenic fragments thereof.

The polypeptides of the invention may be formulated into the vaccine as neutral or salt forms. Pharmaceutically acceptable salts include the acid addition salts (formed with free amino groups of the peptide) and which are formed with inorganic acids such as, for example, hydrochloric or phosphoric acids, or such organic acids such as acetic, oxalic, tartaric and maleic. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine and procaine.

The preparation of vaccines which contain an immunogenic polypeptide(s) as active ingredient(s), is known to one skilled in the art. Typically, such vaccines are prepared as injectables, either as liquid solutions or suspensions; solid forms suitable for solution in, or suspension in, liquid prior to injection may also be prepared. The preparation may also be emulsified, or the protein encapsulated in liposomes.

The vaccine may comprise an attenuated bacterium capable of expressing the polypeptide of the invention.

The active immunogenic ingredients are often mixed with excipients which are pharmaceutically acceptable and compatible with the active ingredient. Suitable excipients are, for example, water, saline, dextrose, glycerol, ethanol, or the like and combinations thereof.

In addition, if desired, the vaccine may contain minor amounts of auxiliary substances such as wetting or emulsifying agents, pH buffering agents, and/or adjuvants which enhance the effectiveness of the vaccine. Examples of adjuvants which may be effective include but are not limited to: aluminum hydroxide, N-acetyl-muramyl-L-threonyl-D-isoglutamine (thr-MDP), N-acetyl-normuramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion. The effectiveness of an adjuvant may be determined by measuring the amount of antibodies directed against an immunogenic polypeptide containing an HBV antigenic sequence resulting from administration of this polypeptide in vaccines which are also comprised of the various adjuvants.

The vaccines are conventionally administered parenterally, by injection, for example, either subcutaneously or intramuscularly. Additional formulations which are suitable for other modes of administration include suppositories and intranasal formulations. Oral formulations may be provided, in particular for administration of attenuated bacterium. For suppositories, traditional binders and carriers may include, for example, polyalkylene glycols or triglycerides; such suppositories may be formed from mixtures containing the active ingredient in the range of 0.5% to 10%, preferably 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%.

The vaccine, for example, comprising an attenuated bacterium is advantageously presented in lyophilised form, for example in capsular form, for oral administration to a patient. Such capsules, tablets and pills for oral administration to a patient may be provided with an enteric coating comprising, for example, Eudragit "S", Eudragit "L", cellulose acetate, cellulose acetate phthalate or hydroxypropylmethyl cellulose. These capsules may be used as such, or alternatively, the lyophilised material may be reconstituted prior to administration, e.g. as a suspension. Reconstitution is advantageously effected in buffer at a suitable pH to ensure the viability of the organisms. In order to protect the attenuated bacteria and the vaccine from gastric acidity, a sodium bicarbonate preparation is advantageously administered before each administration of the vaccine.

### E. Dosage and Administration of Vaccines

The vaccines are administered in a manner compatible with the dosage formulation, and in such amount as will be prophylactically and/or therapeutically effective. The quantity to be administered, which is generally in the range of 5 µg to 250 µg of antigen per dose, depends on the subject to be treated, capacity of the subject's immune system to synthesize antibodies, and the degree of protection desired. Precise amounts of active ingredient required to be administered may depend on the judgement of the practitioner and may be peculiar to each subject

The vaccine may be given in a single dose schedule, or preferably in a multiple dose schedule. A multiple dose schedule is one in which a primary course of vaccination may be with 1-10 separate doses, followed by other doses given at subsequent time intervals required to maintain and or reinforce the immune response, for example, at 1 to 4 months for a second dose, and if needed, a subsequent dose(s) after several months. The dosage regimen will also, at least in part, be determined by the need of the individual and be dependent upon the judgement of the practitioner.

In addition, the vaccine containing the immunogenic HBV antigen(s) may be administered in conjunction with other immunoregulatory agents, for example, immunoglobulins.

### F. Preparation of antibodies against the polypeptides of the invention

The immunogenic polypeptides prepared as described above can be used to produce antibodies, both polyclonal and monoclonal. If polyclonal antibodies are desired, a selected mammal (e.g., mouse, rabbit, goat, horse, etc.) is immunised with an immunogenic polypeptide bearing an HBV epitope(s). Serum from the immunised animal is collected and treated according to known procedures. If serum containing polyclonal antibodies to an HBV epitope contains antibodies to other antigens, the polyclonal antibodies can be purified by immunoaffinity chromatography. Techniques for producing and processing polyclonal antisera are known in the art.

Monoclonal antibodies directed against HBV epitopes in the polypeptides of the invention can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against HBV epitopes can be screened for various properties; i.e., for isotype and epitope affinity.

Antibodies, both monoclonal and polyclonal, which are directed against HBV epitopes are particularly useful in diagnosis, and those which are neutralising are useful in passive immunotherapy. Monoclonal antibodies, in particular, may be used to raise anti-idiotype antibodies. Anti-idiotype antibodies are immunoglobulins which carry an "internal image" of the antigen of the infectious agent against which protection is desired.

Techniques for raising anti-idiotype antibodies are known in the art. These anti-idiotype antibodies may also be useful for treatment of HBV, as well as for an elucidation of the immunogenic regions of HBV antigens.

It is also possible to use fragments of the antibodies described above, for example, Fab fragments.

### G. Immunoassays

Both the polypeptides of the invention, and antibodies produced using the polypeptides of the inventions may be used in immunoassay methods, for example which react immunologically with serum containing HBV antibodies, for example to detect presence of HBV antibodies, or the presence of viral antigens, in biological samples, including for example, blood or serum samples. In particular, the polypeptides and antibodies of the invention may be used to map highly immunogenic regions within the pre-S1 and pre-S2 regions of HBsAg. Design of the immunoassays is subject to a great deal of variation, and a variety of these immunoassays are known in the art. For example, the immunoassay may utilise one viral antigen, for example, a polypeptide of the invention; or alternatively, the immunoassay may use a combination of viral antigens including a polypeptide of the invention. It may also use, for example, an antibody obtained using a method of the invention or a combination of these antibodies directed towards one viral antigen or several viral antigens. Protocols may be based, for example, upon competition, or direct reaction, or sandwich type assays. The immunoassay protocols used may also, for example, use solid supports, or may be by immunoprecipitation. Most assays involve the use of labelled antibody or polypeptide; the labels may be, for example, fluorescent, chemiluminescent, radioactive, or dye molecules. Assays which amplify the signals from the probe are also known; examples of which are assays which utilise biotin and avidin, and enzyme-labelled and mediated immunoassays, such as ELISA assays.

The invention will be described with reference to the following Examples which are intended to be illustrative only and not limiting. The Examples refer to the Figures. Referring to the Figures in more details:

### Description of the Figures

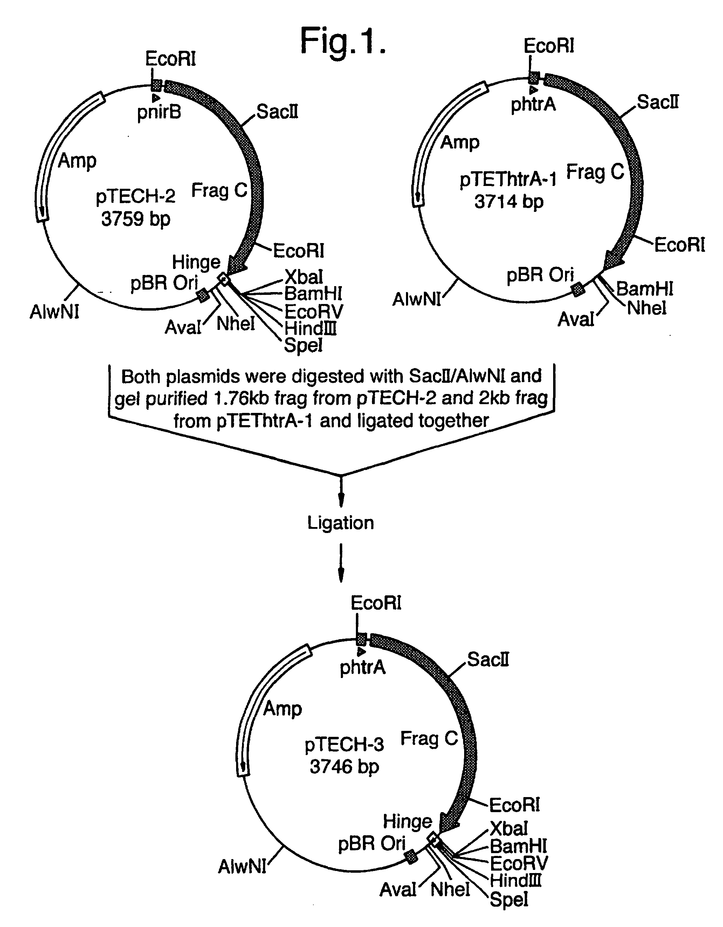

Figure 1 depicts the cloning scheme for plasmid pTECH3
Figure 2
   a) Total Immunoglobulin response, anti-Fragment C, 14 days post prime dose with (■) Fragment C-SΔS1/S2 and (●) Fragment C-BΔS1/S2 purified fusion proteins. Normal mouse serum (non-immunised) response (▲).
   b) Total Immunoglobulin response, anti-Fragment C, 7 days post boost dose with (■) Fragment C-SΔS1/S2 and (●) Fragment C-BΔS1/S2 purified fusion proteins. Normal mouse serum (non-immunised) response (▲).
Figure 2
   a) Total immunoglobulin response, anti-S1 (aa61-81 peptide), 14 days post prime dose with (■) Fragment C-SΔS1/S2 and (●) Fragment C-BΔS1/S2 purified fusion proteins. Normal mouse serum (non-immunised) response (▲).
   b) Total immunoglobulin response, anti-S1 (aa61-81 peptide), 7 days post boost with (■) and (●) Fragment C-BΔS1/S2 purified fusion proteins. Normal mouse serum (non-immunised) response (▲).
Figure 4
   a) Total immunoglobulin response, anti-S1 (aa12-24 peptide), 14 days post prime dose with (■) Fragment C-SΔS1/S2 and (●) Fragment C-BΔS1/S2 purified fusion proteins. Normal mouse serum (non-immunised) response (▲).
   b) Total immunoglobulin response, anti-S1 (aa12-24 peptide), 7 days post boost with (■) Fragment C-SΔS1/S2 and (●) Fragment C-BΔS1/S2 purified fusion proteins. Normal mouse serum (non-immunised) response (▲).
Figure 5
   a) Total immunoglobulin response, anti-S1 (aa21-47 peptide, present in S-pre-S1 particles), 14 days post prime dose with (■) Fragment C-SΔS1/S2 and (●) Fragment C-BΔS1/S2 purified fusion proteins. Normal mouse serum (non-immunised) response (▲).
   b) Total immunoglobulin response, anti-S1 (aa21-47 peptide, present in S-pre-S1 particles), 7 days post boost dose with (■) Fragment C-SΔS1/S2 and (●) Fragment C-BΔS1/S2 purified fusion proteins. Normal mouse serum (non-immunised) response (▲).

### EXAMPLES

### Example 1 - Preparation of expression constructs

Plasmid pTECH-3, the basic expression vector used in these examples, was prepared as shown in Figure 1 from pTECH-2 and pTEThtrA-1 as described in our earlier application PCT/GB95/00196. pTECH-3 comprises a sequence encoding the tetanus toxin fragment C and containing a hinge region, operably linked to the *htrA* promoter.

The constructs described in the table below were constructed as follows:

**Table 1**

| Construct | Hepatitis B sequence |
|---|---|
| pTECH3/S1 | pre S1 _{ayw}21-47aa |
| pTECH3/S2 | pre S2_{ayw} 1-55aa |
| pTECH3/S1/S2 | pre S1 _{ayw} 21-47/preS2 _{ayw} 1-55aa |
| pTECH3/SB | S _{ayw} 120-147aa |
| pTECH3/SΔS1/S2 | pre S1 _{adw} 21-119/S2 _{adw} 1-55aa |
| pTECH3/BΔS1/S2 | pre S1 _{adw} 42-119/S2 _{adw} 1-55aa |
| pTECH3/WS1/S2 | pre S1 _{adw} 1-119/S2 _{adw} 1-55aa |

Plasmid pMBdS1RN/44 (containing the *ayw* hepatitis B pre-S1(20-47)/S gene) was used as template for constructing pTECH3/S1, pTECH3/SB and pTECH3/S1/S2. pMByS2/8 (containing the *ayw* hepatitis B pre-S2(1-55)/S gene) was used as the template for pTECH3/S2 and pTECH3/S1/S2 and pRIT12793 (containing the entire *adw* hepatitis B pre-S1/S2 gene in pBR322) was used for pTECH3/SΔS1/S2, pTECH3/BΔS1/S2, and pTECH3/WS1/S2.

The following pairs of primers were used to PCR clone, using standard conditions, the hepatitis B pre-S1/S2 sequences for insertion into pTECH3:

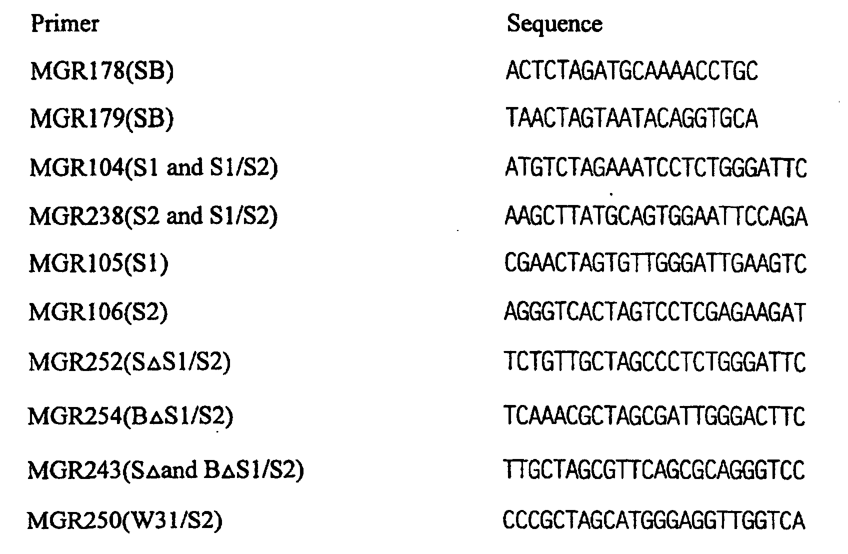

Each PCR fragment was digested with restriction enzymes, gel purified and ligated with a 3.76 kbp pTECH3 fragment. The first four constructs were made by digesting the pTECH3 plasmid with XbaI/SpeI/CIAP to produce the 3.76 kbp fragment. The appropriate HBV PCR fragment which had been pre-cut with NheI was then ligated to the 3.76 kbp fragment. The last two constructs were made by digesting pTECH3 plasmid with XbaI/CIAP to produce the 3.76 kbp fragment which was then ligated with the appropriate HBV PCR product which had been pre-cut with NheI.

### Example 2 - Expression of polypeptides and Western Blotting

Plasmids were transformed into *E. coli* strain HB 101 using standard techniques. Expression of fusion polypeptides was induced by heat stress at 37°C. Expression was tested by western blotting *E*. *coli* cellular extracts with anti-tetanus toxin fragment C antibody and specific antibodies for each insert sequence (see Table 2).

### Example 3 - Production of polyclonal antibodies against polypeptide constructs

The fusion polypeptides produced in Example 2 from plasmids pTECH3/sΔS1/S2 and pTECH3/BΔS1/S2 were purified from *E. coli* cellular extracts by affinity chromatography using anti-tetanus toxin fragment C as the ligand immobilised to the sepharose 4B column. Purified proteins were prepared for injection and administered to mice (B10, female, 6-8 weeks old) using standard techniques and as detailed below.

### Schedule

| | |
|---|---|
| Group 1 | Immunise mice (prime dose) intra-peritoneal I/P with 5 µg purified Frag C-SΔS1/S2 fusion protein. |
| | Sample bleed 14 days post prime dose. |
| | Immunise mice (boost dose) I/P with 5 µg purified Frag C-SΔS1/S2 fusion protein, 21 days post prime dose Bleed-out mice 7 days post boost dose. |
| | |
| Group 2 | Immunise mice (prime dose) intra-peritoneal I/P with 5 µg purified Frag C-BΔS1/S2 fusion protein. |
| | Sample bleed 14 days post prime dose. |
| | Immunise mice (boost dose) I/P with 5 µg purified Frag C-BΔS1/S2 fusion protein, 21 days post prime dose Bleed-out mice 7 days post boost dose. |

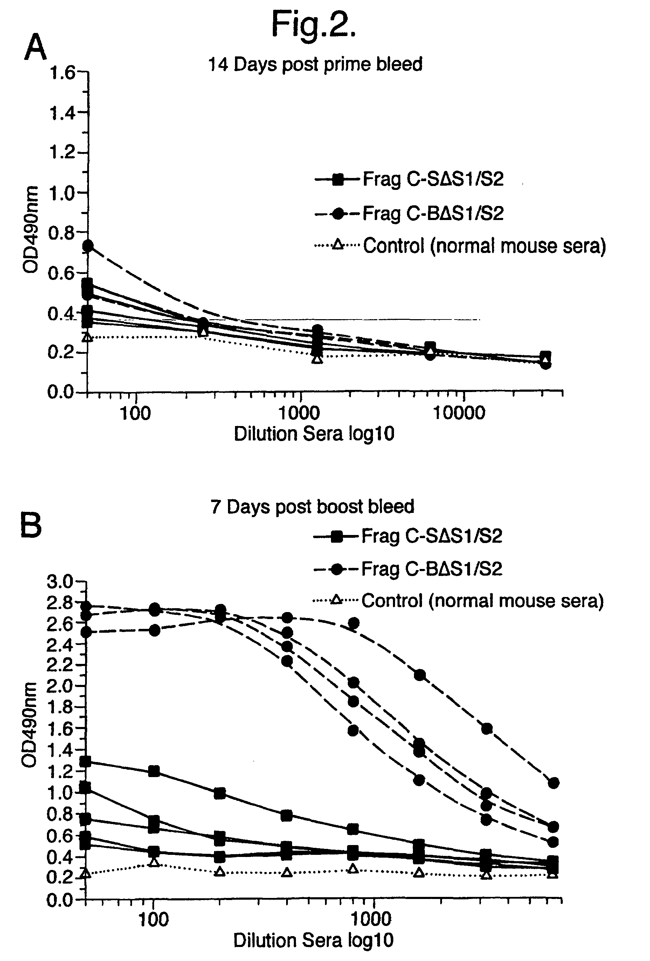

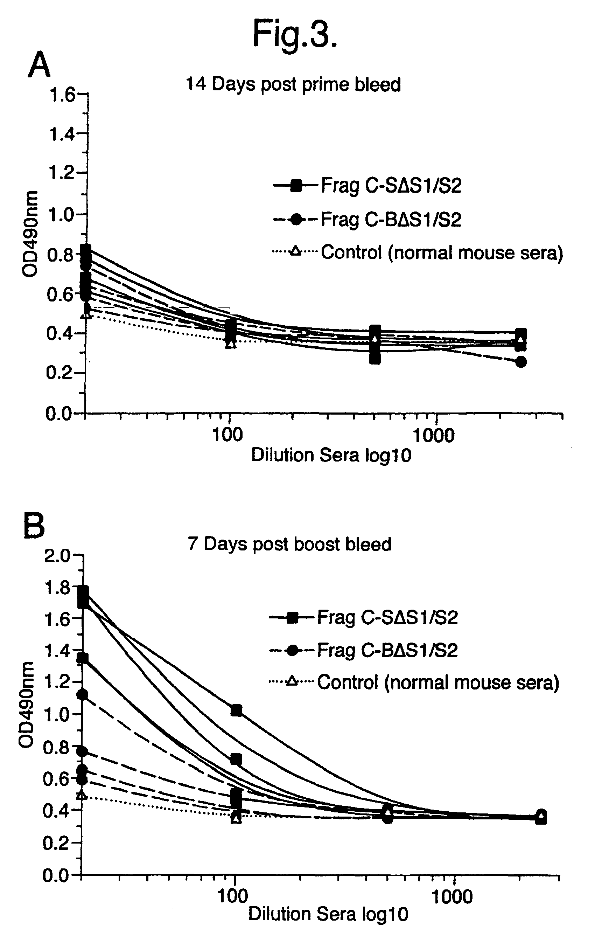

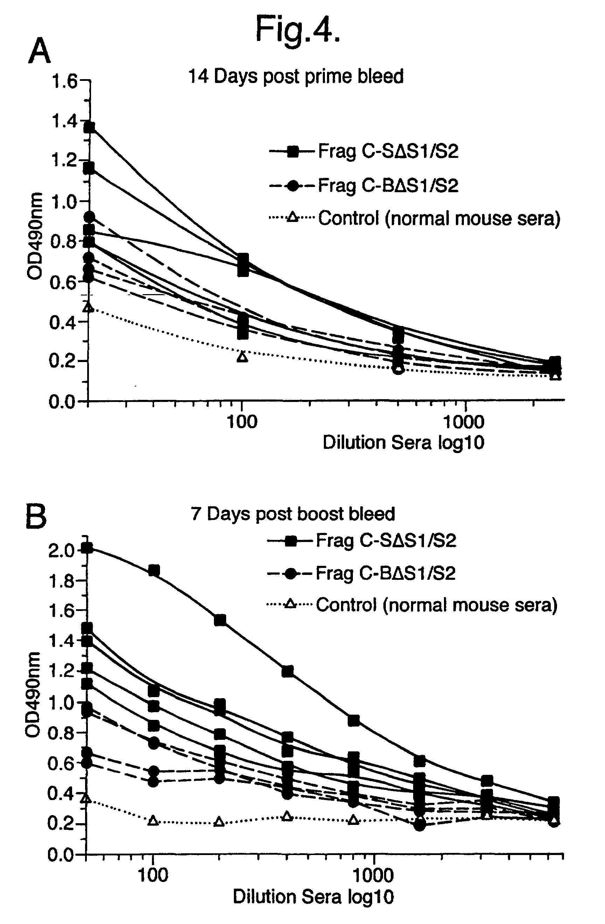

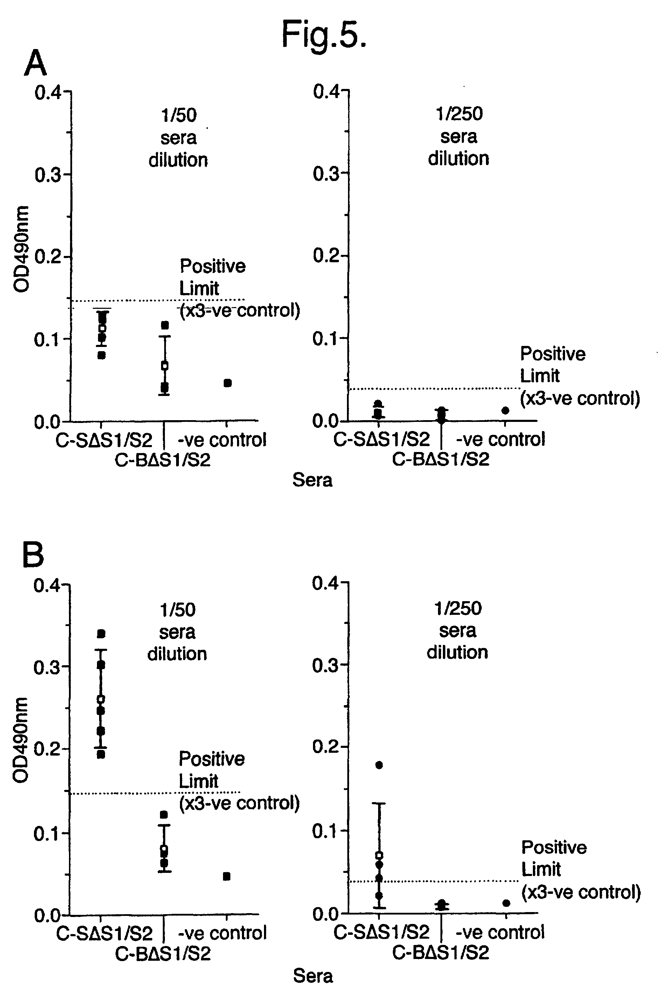

Total antibody responses were determined by ELISA against purified fragment C, pre-S1 (aa61-81), pre-S2 (aa12-24) peptides and pre-S1 (aa21-47) contained within S-S1 particles. Responses are illustrated in Figures 2, 3, 4 and 5 respectively.

In summary, responses were detected to both pre-S 1 and pre-S2 components of the fusion proteins, and the fragment C carrier protein.

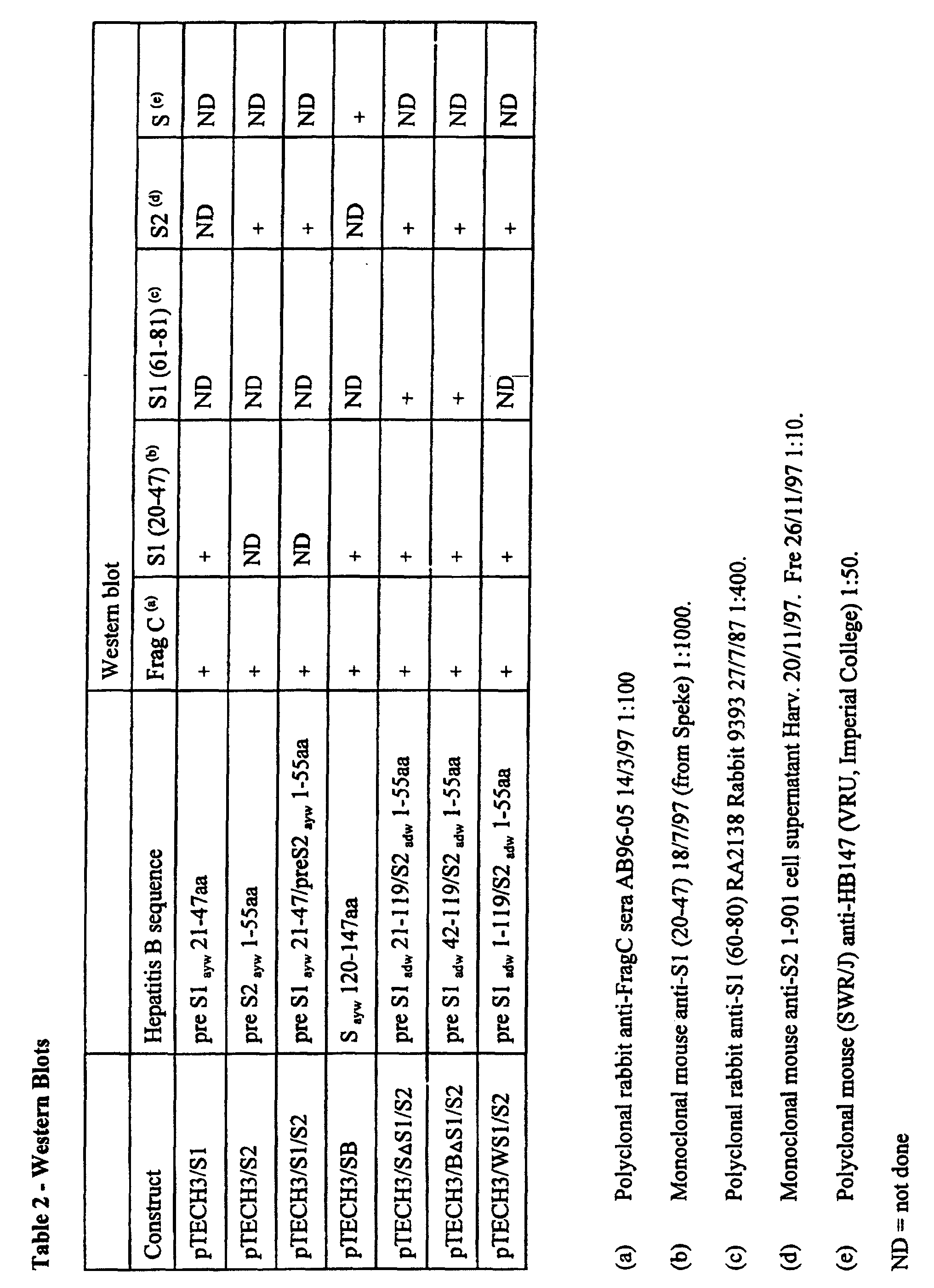

## Claims

1. A polypeptide comprising
(i) tetanus toxin fragment C or a fragment thereof of at least 6 amino acids, fused to
(ii) the pre-S1 region of hepatitis B virus (HBV) or a fragment thereof of at least 6 amino acids, and/or the pre-S2 region of HBV or a fragment thereof of at least 6 amino acids,
wherein the polypeptide induces antibody that recognises the pre-S1 and/or pre-S2 region of HBV.

2. A polypeptide according to claim 1 which comprises a fragment of tetanus toxin fragment C of at least 100 amino acids.

3. A polypeptide according to claim I which comprises full length fragment C.

4. A polypeptide according to claim 1, 2 or 3 which comprises a fragment of the pre-S1 region of at least 20 amino acids and/or a fragment of the pre-S2 region of at least 20 amino acids.

5. A polypeptide according to any one of the preceding claims including amino acids 20 to 47 or 21 to 47 of the pre-S1 region.

6. A polypeptide according to any one of the preceding claims including amino acids 1 to 26 or 14 to 32 of the pre-S2 region.

7. A polynucleotide encoding a polypeptide according to any one of the preceding claims.

8. A vector comprising a polynucleotide according to claim 7 operably linked to a regulatory sequence.

9. A vector according to claim 8 wherein said regulatory sequence comprises an *htrA* promoter sequence.

10. A host cell comprising a vector according to claim 8 or 9.

11. A host cell according to claim 10 which is a bacterium.

12. A vaccine composition comprising a polypeptide according to any one of claims 1 to 6, a polynucleotide according to claim 7 or a vector according to claim 8 or 9, together with a pharmaceutically acceptable carrier to diluent.

13. A polypeptide according to any one of claims 1 to 6, a polynucleotide according to claim 7 or a vector according to claim 8 or 9, for use in a method of treating or preventing HBV infection in a human or animal.

14. A polypeptide according to any one of claims 1 to 6, a polynucleotide according to claim 7 or a vector according to claim 8 or 9 for use in a method for producing antibodies in a mammal which recognise epitopes within the pre-S1 and/or pre-S2 region of HBV.

15. Use of a polypeptide according to any one of claims 1 to 6, a polynucleotide according to claim 7 or a vector according to claim 8 or 9 for the manufacture of a medicament for treating or preventing HBV infection in a human or animal.

## Patentansprüche

1. Polypeptid, umfassend:
(i) Tetanustoxinfragment C oder ein Fragment davon aus mindestens 6 Aminosäuren, kondensiert an
(ii) den Prä-S1-Bereich des Hepatitis B Virus (HBV) oder ein Fragment davon aus mindestens 6 Aminosäuren und/oder den Prä-S2-Bereich von HBV oder ein Fragment davon aus 6 Aminosäuren,
wobei das Polypeptid Antikörper einschließt, der den Prä-S1- und/oder Prä-S2-Bereich von HBV erkennt.

2. Polypeptid nach Anspruch 1, das ein Fragment von Tetanustoxinfragment C aus mindestens 100 Aminosäuren umfasst.

3. Polypeptid nach Anspruch 1, das das Fragment C in vollständiger Länge umfasst.

4. Polypeptid nach Anspruch 1, 2 oder 3, das ein Fragment des Prä-S1-Bereichs aus mindestens 20 Aminosäuren und/oder ein Fragment des Prä-S2-Bereichs aus mindestens 20 Aminosäuren umfasst.

5. Polypeptid nach einem der vorhergehenden Ansprüche, das Aminosäuren 20 bis 47 oder 21 bis 47 des Prä-S1-Bereichs einschließt.

6. Polypeptid nach einem der vorhergehenden Ansprüche, das Aminosäuren 1 bis 26 oder 14 bis 32 des Prä-S2-Bereichs einschließt.

7. Polynukleotid, das ein Polypeptid nach einem der vorhergehenden Ansprüche kodiert.

8. Vektor, der ein Polynukleotid nach Anspruch 7 umfasst, das in Wirkbeziehung an eine Regulatorsequenz gekoppelt ist.

9. Vektor nach Anspruch 8, bei dem die Regulatorsequenz eine *htrA*-Promotersequenz umfasst.

10. Wirtszelle, die einen Vektor nach Anspruch 8 oder 9 umfasst.

11. Wirtszelle nach Anspruch 10, die ein Bakterium ist.

12. Impfstoffzusammensetzung, die ein Polypeptid nach einem der Ansprüche 1 bis 6, ein Polynukleotid nach Anspruch 7 oder einen Vektor nach Anspruch 8 oder 9 zusammen mit einem pharmazeutisch annehmbaren Träger als Verdünnungsmittel umfasst.

13. Polypeptid nach einem der Ansprüche 1 bis 6, Polynukleotid nach Anspruch 7 oder Vektor nach Anspruch 8 oder 9 zur Verwendung in einem Verfahren zum Behandeln oder Verhindern der HBV-Infektion bei einem Menschen oder Tier.

14. Polypeptid nach einem der Ansprüche 1 bis 6, Polynukleotid nach Anspruch 7 oder Vektor nach Anspruch 8 oder 9 zur Verwendung in einem Verfahren zum Produzieren von Antikörpern in einem Säugetier, das Epitope innerhalb des Prä-S1- und/oder Prä-S2-Bereichs von HBV erkennt.

15. Verwendung eines Polypeptids nach einem der Ansprüche 1 bis 6, eines Polynukleotids nach Anspruch 7 oder eines Vektors nach Anspruch 8 oder 9 zur Herstellung eines Medikaments zum Behandeln oder Verhindern der HBV-Infektion bei einem Menschen oder Tier.

## Revendications

1. Polypeptide comprenant
(i) le fragment C de toxine tétanique ou l'un de ses fragments d'au moins 6 acides aminés, fusionné à
(ii) la région pré-S1 du virus de l'hépatite B (HBV) ou l'un de ses fragments d'au moins 6 acides aminés, et/ou la région pré-S2 du virus de l'hépatite B (HBV) ou l'un de ses fragments d'au moins 6 acides aminés,
le polypeptide induisant un anticorps qui reconnaît la région pré-S1 et/ou pré-S2 de HBV.

2. Polypeptide selon la revendication 1, qui comprend un fragment du fragment C de toxine tétanique d'au moins 100 acides aminés.

3. Polypeptide selon la revendication 1, qui comprend le fragment C de longueur totale.

4. Polypeptide selon la revendication 1, 2 ou 3, qui comprend un fragment de la région pré-S1 d'au moins 20 acides aminés et/ou un fragment de la région pré-S2 d'au moins 20 acides aminés.

5. Polypeptide selon l'une quelconque des revendications précédentes incluant les acides aminés 20 à 47 ou 21 à 47 de la région pré-S1.

6. Polypeptide selon l'une quelconque des revendications précédentes incluant les acides aminés 1 à 26 ou 14 à 32 de la région pré-S2.

7. Polynucléotide codant un polypeptide selon l'une quelconque des revendications précédentes.

8. Vecteur comprenant un polynucléotide selon la revendication 7, lié de façon opérationnelle à une séquence régulatrice.

9. Vecteur selon la revendication 8, dans lequel ladite séquence régulatrice comprend une séquence de promoteur *htrA*.

10. Cellule hôte comprenant un vecteur selon la revendication 8 ou 9.

11. Cellule hôte selon la revendication 10, qui est une bactérie.

12. Composition vaccinale comprenant un polypeptide selon l'une quelconque des revendications 1 à 6, un polynucléotide selon la revendication 7 ou un vecteur selon la revendication 8 ou 9, avec un support ou un diluant pharmaceutiquement acceptable.

13. Polypeptide selon l'une quelconque des revendications 1 à 6, polynucléotide selon la revendication 7 ou vecteur selon la revendication 8 ou 9, destiné à être utilisé dans une méthode de traitement ou de prévention d'une infection par HBV chez un humain ou un animal.

14. Polypeptide selon l'une quelconque des revendications 1 à 6, polynucléotide selon la revendication 7 ou vecteur selon la revendication 8 ou 9, destiné à être utilisé dans une méthode pour produire chez un mammifère des anticorps qui reconnaissent des épitopes dans la région pré-S1 et/ou pré-S2 de HBV.

15. Utilisation d'un polypeptide selon l'une quelconque des revendications 1 à 6, d'un polynucléotide selon la revendication 7 ou d'un vecteur selon la revendication 8 ou 9, pour la fabrication d'un médicament pour traiter ou prévenir une infection par HBV chez un humain ou un animal.
